# EUROPÄISCHE PATENTSCHRIFT

(11) **EP 0 419 872 B1**
(45) Veröffentlichungstag und Bekanntmachung des Hinweises auf die Patenterteilung: **26.04.1995**
(21) Anmeldenummer: 90116534.0
(22) Anmeldetag: 29.08.1990
(51) Int. Cl.: A61B 17/22

(54) **Vorrichtung zur räumlichen Ortung und zur Zerstörung von körperinneren Objekten**
Device for spatial location and destruction of objects in the body
Dispositif pour la localisation spatiale et la destruction des objets dans le corps

(30) Priorität: 28.09.1989 DE 3932364
(43) Veröffentlichungstag der Anmeldung: 03.04.1991
(73) Patentinhaber: Richard Wolf GmbH, 75438 Knittlingen (DE)
(72) Erfinder: Belikan, Thomas, D-7134 Knittlingen (DE); Krauss, Werner, D-7134 Knittlingen (DE); Wurster, Helmut, D-7519 Oberderdingen (DE)
(74) Vertreter: Wilcken, Thomas, Dipl.-Ing.

(56) Entgegenhaltungen:
- EP-A- 0 169 311
- FR-A- 2 591 467
- FR-A- 2 608 913
- US-A- 3 387 604
- Biliary Lithotripsy (Eds. J.T. Ferrucci et al.), Adapted from the Proceedings of the First International Symposium on Biliary Lithotripsy, July 11-13, 1988, Seiten 287-291; H. Wurster: "Richard Wolf: Piezolith 2300"

## Beschreibung

Die Erfindung geht aus von einer Vorrichtung zur räumlichen Ortung und zur Zerstörung von körperinneren Objekten, insbesondere von in Körperhöhlen befindlichen Konkrementen, gemäß dem Oberbegriff des Patentanspruches 1.

Eine derartige Vorrichtung ist in der FR-A-2 591 467 beschrieben. Sie besteht aus einem elektroakustischen Therapiewandler zur Erzeugung von fokussiert auf das jeweilige Objekt auszusendenden Stoßwellen für die Therapie und aus mindestens einem mit dem Therapiewandler verbunden B-Scanner als Ortungswandler für die Ortung des Objektes, wobei der Ortungswandler relativ zum Therapiewandler auf einer vorgegebenen Kreisbahn außerhalb des Therapiewandlers und mit konstantem Abstand (konfokal) zum Stoßwellenfokus des Therapiewandlers verstellbar ist, um je nach Stellung des Ortungswandlers B-Bilder in verschiedenen Schnittebenen und aus verschiedenen Blickwinkeln erzeugen und darstellen zu können.

Mit B-Scannern arbeitende Ultraschall-Ortungssysteme dieser bekannten Vorrichtungen bzw. Lithotriptoren vermitteln dem Arzt noch zu wenig Informationen über Größe, Volumen und Oberfläche des zu therapierenden Konkrementes. Besonders bei der Behandlung von großen Gallenblasensteinen ist aber eine einwandfreie Beurteilung der Form und Abmessungen des Steines von großer Bedeutung, und zwar auch während der Therapie, da einerseits eine größtmögliche Steindesintegration angestrebt werden muß und andererseits auch die Möglichkeit bestehen soll, exakte Informationen über den jeweils erreichten Therapieerfolg anhand der auf einem Monitor dargestellten B-Bilder zu gewinnen und erforderlichenfalls zu dokumentieren.

Aus der FR-A-2 591 467 Fig. 1 und 2 ist es auch bekannt, den Ortungswandler und den Therapiewandler auf gleicher Achse anzuordnen und den Ortungswandler relativ zum Therapiewandler axial auf dieser Achse und um diese Achse verstellen zu können. Hierdurch lassen sich zwar B-Bilder in verschiedenen Schnittebenen erzeugen, jedoch ist eine Betrachtung des Konkrementes aus verschiedenen Blickwinkeln nicht möglich, so daß diese Lösung in bezug auf den Informationsgehalt des B-Bildes noch nicht optimal ist. Insofern ist diejenige Lösung nach dieser Druckschrift besser, bei welcher der Ortungswandler außen am Umfang des Therapiewandlers angeordnet ist und auf einer Kreisbahn konzentrisch zur Längsachse des Therapiewandlers verstellt werden kann, weil dann je nach Stellung des Ortungswandlers das zu zerstörende Konkrement unter verschiedenen Blickwinkeln aufgenommen und betrachtet werden kann.

Aber auch die letztgenannte Lösung hat gewisse Nachteile im Zusammenhang mit der Zielgenauigkeit, weil die Schallfelder beider Wandler nicht gleiche Wege und Medien durchlaufen und deshalb nicht immer gewährleistet ist, daß ein beispielsweise auf dem Bildschirm des zum Ortungssystem gehörenden Monitors angebrachtes, den Ort des Stoßwellenfokus im B-Bild markierendes Zielkreuz tatsächlich die richtige Position des Fokus im Verhältnis zum abgebildeten Konkrement darstellt.

Weiterhin ist es bei der letztgenannten Lösung nicht möglich, den B-Scanner in einem optimalen Winkel zur Achse des Therapiewandlers anzuordnen. Dieser Winkel ist nämlich aufgrund des extern am Therapiewandler angebrachten Ortungswandlers zu groß, so daß die Ausrichtung der sektorförmigen Abtastebene des Ortungswandlers auf das Konkrement aus baulichen und anatomischen Gründen problematisch oder sogar nicht möglich ist.

Günstiger ist es deshalb, zwei auf den Stoßwellenfokus des Therapiewandlers ausgerichtete Ortungswandler unter einem spitzen Winkel von beispielsweise 15 ° zur Längsachse des Therapiewandlers auf oder in diesem anzuordnen, wie es aus Biliary Lithotripsy (Eds. J. T. Ferucci et al.), Adapted from the Proceeding of the First International Symposium on Biliary Lithotripsy, July 11-13, 1988, Seiten 287-291; H. Wurster: "Richard Wolf: Piezolith 2300" bekannt ist. Obwohl hierbei durch Rotation des jeweiligen Ortungswandlers um 90° um seine eigene Achse das Konkrement aus verschiedenen Blickwinkeln dargestellt und betrachtet werden kann, ergibt sich der Nachteil, daß das Konkrement nicht aus verschiedenen Blickwinkeln durch Verstellung der Ortungswandlerachse dargestellt und betrachtet werden kann.

Dementsprechend besteht die Aufgabe der Erfindung darin, die Dynamik und damit auch den Informationsgehalt der vom Ortungswandler bzw. von den Ortungswandlern erzeugten B-Bilder im Vergleich zu vorbekannten Lösungen wesentlich zu verbessern.

Die Lösung dieser Aufgabe ist in dem Kennzeichen des Anspruchs 1 angegeben.

Mit einer derartig ausgestalteten Vorrichtung ist der Informationsgehalt über das zu therapierende Konkrement wesentlich verbessert, da sich so eine Vielzahl von Bildern erzeugen läßt, die das Konkrement in verschiedenen Schnittebenen und aus verschiedenen Blickwinkeln darstellen. Bei Wiedergabe dieser Bilder, beispielsweise auf dem Bildschirm eines dem Ortungssystem zugehörenden Monitors, ergibt sich zugleich der Vorteil, daß ein auf dem Bildschirm desselben angebrachtes, den Ort des Stoßwellenfokus im B-Bild markierendes Zielkreuz die richtige Position des Fokus im Verhältnis zum abgebildeten Konkrement darstellt. Dies ergibt sich daraus, daß die Schallfelder beider Wandler gleiche Wege und Medien durchlaufen, so daß unterschiedliche Brechungen der Wellenfronten der beiden Schallfelder und damit daraus sich herleitende eventuelle Abbildungsfehler ausgeschlossen sind. Es kann so eine Verbesserung der Zielgenauigkeit und Präzision der Therapie erreicht werden, da das Schallfeld des Therapiewandlers die gleichen Bedingungen vorfindet wie das des Ortungswandlers.

Konstruktiv einfache und für den Ortungsvorgang sichere Lösungen ergeben sich, wenn die Bahn, in Aufsicht auf den Therapiewandler gesehen, als Gerade verläuft, wobei die Bahn die Längsachse desselben auch schneiden kann.

Die Bahn kann aber auch als Kreisring oder Kreisringabschnitt um die Längsachse des Therapiewandlers verlaufen.

Nach einer bevorzugten Ausführung kann der Ortungswandler entlang seiner Bahn motorisch verstellbar sein, was zu einer weiteren Steigerung des Informationsgehaltes über das zu therapierende Konkrement führen kann, wenn die so aus verschiedenen Winkeln erhaltenen "bewegten" Abbildungen desselben mit Hilfe eines zusätzlichen Rechners derart verarbeitet werden, daß eine überlagerte Wiedergabe der Einzelbilder in einer Art Compound-Scan-Verfahren ermöglicht wird.

Die Zuordnung des Ortungswandlers zu dem Therapiewandler kann vorteilhaft so erfolgen, daß der Therapiewandler im Bereich der Bahn ausgespart ist und daß der Ortungswandler in die Aussparung eingreift oder diese durchgreift. Dabei kann der Ortungswandler sowohl um seine Längsachse verdrehbar, als auch in dieser axial verstellbar sein, wodurch es möglich ist, einerseits in jeder Winkelstellung des Ortungswandlers zur Längsachse des Therapiewandlers B-Bilder in verschiedenen, entsprechend winkelversetzten Schnittebenen zu erzeugen und andererseits eine Anpassung der Position des Ortungswandlers entsprechend den anatomischen Gegebenheiten des Körpers des jeweils zu behandelnden Patienten vorzunehmen.

Schließlich kann eine einfache Austauschbarkeit des Ortungswandlers, beispielsweise zur Anpassung der Vorrichtung an die jeweiligen Applikationen, dadurch ermöglicht werden, daß der Kopf des Ortungswandlers durch Steckverbindung lösbar und auswechselbar gehaltert wird.

Die erfindungsgemäße Vorrichtung ist nachfolgend anhand von in der Zeichnung dargestellten Ausführungsbeispielen näher erläutert. Es zeigen:
- Figur 1: einen Querschnitt durch einen Therapiewandler mit einem längs einer zentralen Geraden durch diesen verschwenkbaren Ortungswandler,
- Figur 2: eine Draufsicht auf den Therapiewandler nach Figur 1,
- Figur 3: eine axonometrische Darstellung des Therapiewandlers nach den Figuren 1 und 2,

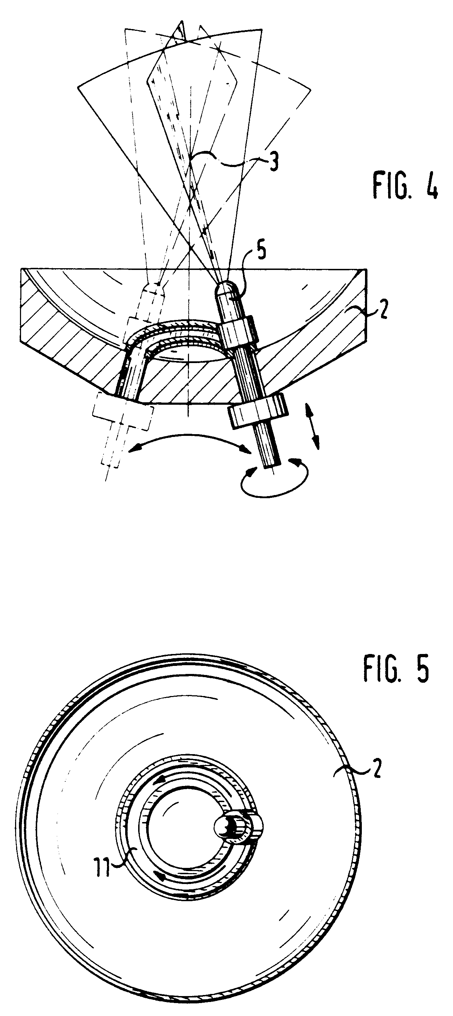

- Figur 4: einen Querschnitt durch einen Therapiewandler mit einem um die Achse desselben konzentrisch führbaren Ortungswandler,
- Figur 5: eine Draufsicht auf den Therapiewandler nach Figur 4,
- Figur 6: eine axonometrische Darstellung des Therapiewandlers nach den Figuren 4 und 5,
- Figur 7: eine schematisiertes Ausführungsbeispiel für die motorische Erzeugung der Verstellbewegungen des Ortungswandlers,
- Figur 8: ein Ausführungsbeispiel für ein schnellwechselbares Wandlerelement,

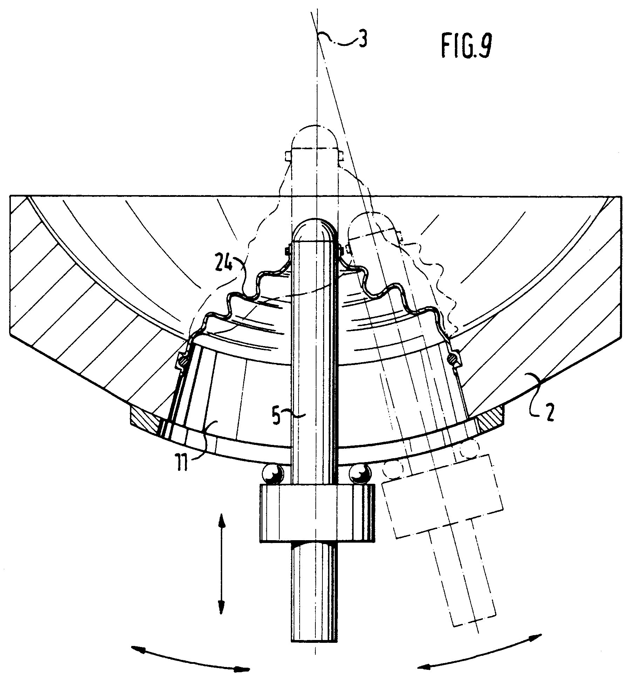

- Figur 9: ein Ausführungsbeispiel für die Abdichtung eines Ortungswandlers gegenüber der Wandler-Kalotte des Therapiewandlers.

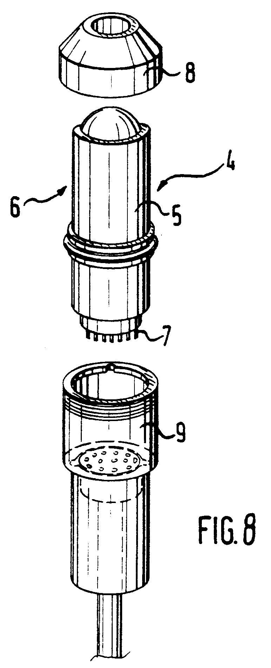

Der erfindungsgemäße, als selbstfokussierender Wandler ausgebildete Therapiewandler 2 hat bei allen dargestellten Vorrichtungen die Form einer Kugelkalotte, in der einzelne, nicht näher dargestellte piezoelektrische Wandlerelemente so angeordnet sind, daß ihre Strahlung auf den Stoßwellenfokus 3 des Wandlers 2 gerichtest ist. Der Polbereich der Kalottenfläche des Therapiewandlers 2 ist von Wandlerelementen freigehalten, so daß über diesem Bereich ein Schallschatten mit kegelförmiger Umfangsform entsteht. In dem genannten Polbereich ist ein Ortungssystem 4 integriert. Das Ortungssystem 4 besteht aus einem als B-Scanner wirksamen Ortungswandler 5, der gemäß Figur 8 als mit einem Steckanschluß 7 versehenes Steckelement 6 ausgebildet sein kann. Dabei ist das Steckelement 6 mittels eines Schraubringes 8 unter flüssigkeitsdichter Abdichtung in einer Steckfassung 9 festgelegt.

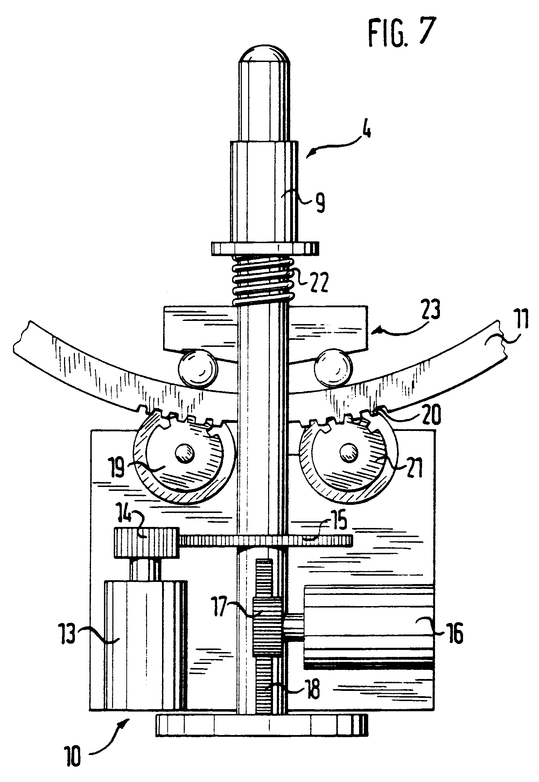

Die Steckfassung 9 ist Bestandteil einer Schlitteneinheit 10, die in einer schlitzförmigen Aussparung 11 in dem kalottenförmigen Therapiewandler 2 geführt ist. Die Schlitteneinheit 10 ist entsprechend Figur 10 als Antriebseinheit ausgeführt, die Antriebsmittel für eine Verschwenkung, Verdrehung und axiale Verschiebung relativ zum Pol der Kugelkalotte des Therapiewandlers 2 sowie Rückmeldemittel für die jeweilige Positionserfassung umfaßt. Entsprechend der in Figur 7 schematisiert dargestellten Ausführung bestehen die Antriebsmittel für die Verdrehung aus einem regelbaren Motor 13, der über ein Zahnradgetriebe 14, 15 die Steckfassung 9 und damit den Ortungswandler 5 um seine Achse verdreht. Ebenso umfassen die Antriebsmittel für die axiale Verschiebung einen regelbaren Motor 16, der über Ritzel 17 und Zahnstange 18 auf die Steckfassung 9 einwirkt, die dementsprechend in geeigneter Weise in der Schlitteneinheit 10 geführt bzw. gelagert ist. Die Verschwenkung des Ortungswandlers 5 erfolgt durch einen nicht gezeigten regelbaren Motor, der mit einem Ritzel 19 beispielsweise nach dem Triebstocksystem in einer Verzahnung 20 an der Kugelkalotte des Therapiewandlers 2 eingreift. Dabei ist die Verzahnung 20 jeweils in ihrem Verlauf der jeweiligen Form der den Ortungswandler 5 führenden Aussparung 11 angepaßt ausgeführt.

Die genannten Motoren können Schrittmotoren sein, welche durch Steuersignale in Impulsform gesteuert werden. Die Positionsrückmeldung kann jeweils durch ein beispielhaft in Figur 7 dargestelltes, als Weggeber fungierendes Potentiometer 21 erfolgen.

Für eine spiel freie Führung des Ortungswandlers 5 sorgt eine Feder 22, die die an der Außenfläche der Kugelkalotte des Therapiewandlers 2 geführte Schlitteneinheit 10 und einen an der Innenfläche angeordneten Schlittenteil 23 gegeneinander verspannt.

Die Abdichtung des Ortungswandlers 5 gegenüber der Kugelkalotte des Therapiewandlers 2 erfolgt mittels eines Faltenbalges 24, der die Verstellbahn des Ortungswandlers 5 bildende Aussparung 11 abdeckt, und der an dem Steckelement 6 bzw. der Steckfassung 9 des Ortungswandlers 5 auch unter Drehung dichtend befestigt ist.

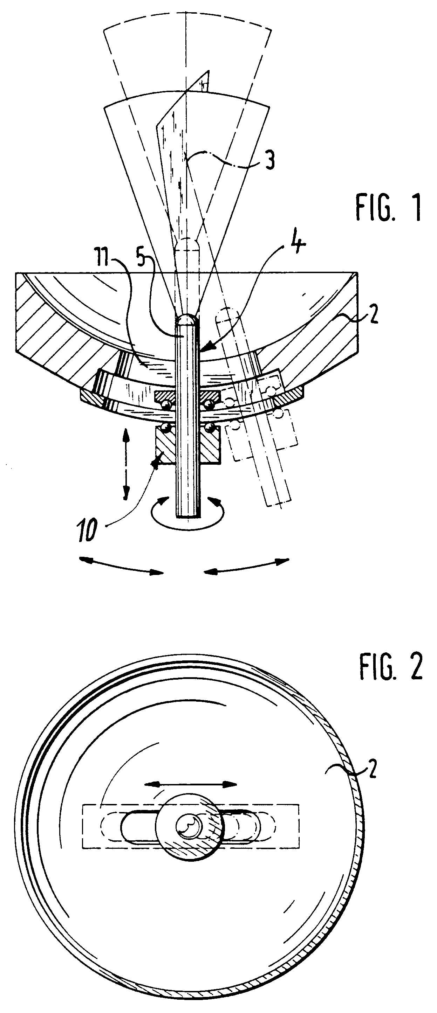

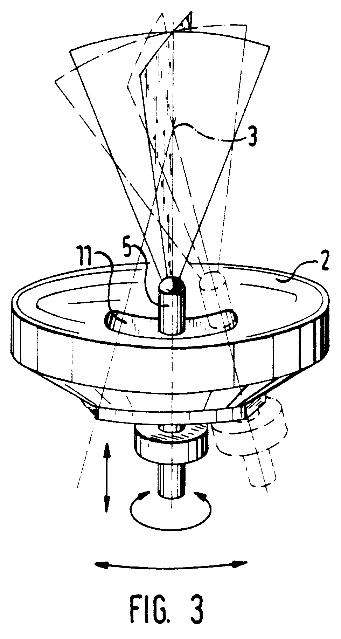

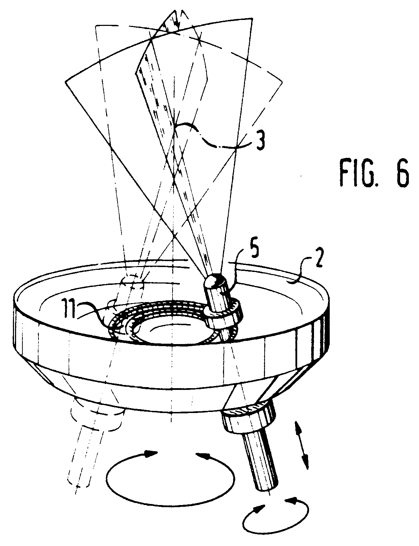

Der in den Ausführungsbeispielen angewendete B-Scanner weist ein fächerförmiges Schallfeld auf. Das entsprechend Figuren 1, 2 und 3 aufgebaute Ortungssystem 4 ist daher in der Lage, den georteten Gegenstand durch Drehung des Ortungswandlers 5 um seine Achse in Mehrfachschnittebenen darzustellen, und durch die Schwenkbewegung deren Winkellage zu verändern. In Figur 3 ist die Ausbildung der Schallfeldfächer bei einer Drehung des Ortungswandlers 5 um 90 Grad in Vollinien-Darstellung gezeigt, während die gestrichelte Darstellung diesen Schallfeldfächer in einer Schwenkstellung des Ortungswandlers 5 wiedergibt. Bei der Vorrichtung nach Figur 6 ist neben der Darstellung des georteten Gegenstandes in Mehrfachschnittebenen dessen Betrachtung von verschiedenen Standorten aus, jedoch unter einheitlicher Winkellage möglich.

## Patentansprüche

1. Vorrichtung zur räumlichen Ortung und zur Zerstörung von körperinneren Objekten, bestehend aus einem elektroakustischen selbstfokussierenden und als Kugelkalotte ausgebildeten Therapiewandler (2) zur Erzeugung von fokussiert auf das jeweilige Objekt auszusendenden Stoßwellen und aus mindestens einem mit dem Therapiewandler verbundenen B-Scanner als Ortungswandler (5) für die Ortung des Objektes, wobei der Ortungswandler (5) relativ zum Therapiewandler (2) auf einer vorgegebenen Bahn und mit konstantem Abstand (konfokal) zum Stoßwellenfokus (3) des Therapiewandlers verstellbar ist, um je nach Stellung des Ortungswandlers B-Bilder in verschiedenen Schnittebenen und aus verschiedenen Blickwinkeln erzeugen und darstellen zu können, dadurch gekennzeichnet, daß die vorgegebene Verstellbahn des Ortungswandlers (5) durch eine in der Kugelkalotte des Therapiewandlers (2) vorgesehene mechanische schlitzförmige Aussparung (11) für den vom Stoßwellenschallfeld umgebenen Ortungswandler (5) gebildet ist.

2. Vorrichtung nach Anspruch 1, dadurch gekennzeichnet, daß die Verstellbahn für den Ortungswandler - in Achsrichtung des Therapiewandlers auf die Abstrahlfläche betrachtet - als Gerade ausgebildet ist.

3. Vorrichtung nach einem der Ansprüche 1 oder 2, dadurch gekennzeichnet, daß die Verstellbahn (11) die Längsachse des Therapiewandlers (2) schneidet.

4. Vorrichtung nach Anspruch 1, dadurch gekennzeichnet, daß die Verstellbahn als Kreisring oder Kreisringabschnitt um die Längsache des Therapiewandlers (2) ausgebildet ist.

5. Vorrichtung nach Anspruch 1, dadurch gekennzeichnet, daß der Ortungswandler (5) motorisch entlang der Verstellbahnverstellbar ist.

6. Vorrichtung nach Anspruch 1, dadurch gekennzeichnet, daß der Ortungswandler (5) um seine Längsachse verdrehbar ist.

7. Vorrichtung nach Anspruch 1, dadurch gekennzeichnet, daß der Ortungswandler (5) axial verstellbar ist.

8. Vorrichtung nach Anspruch 1, dadurch gekennzeichnet, daß der Kopf des Ortungswandlers (5) lösbar und auswechselbar ist.

## Claims

1. A device for the three-dimensional locating and for the destruction of objects within the body, consisting of an electroacoustic self-focussing therapy transducer (2), constructed as a spherical calotte, for the production of shock waves which are to be transmitted in a focussed manner onto the respective object, and of at least one B-scanner, connected with the therapy transducer, as locating transducer (5) for the locating of the object, in which the locating transducer (5) is adjustable relative to the therapy transducer (2) on a given path and at a constant distance (confocal) from the shock wave focus (3) of the therapy transducer, in order to be able to produce and represent, depending on the position of the locating transducer, B-images in various sectional planes and from various angles of view, characterised in that the given adjustment path of the locating transducer (5) is formed by a mechanical slit-shaped recess (11), provided in the spherical calotte of the therapy transducer (2), for the locating transducer (5) which is surrounded by the shock wave sound field.

2. A device according to Claim 1, characterised in that the adjustment path for the locating transducer - viewed in axial direction of the therapy transducer onto the reflecting surface - is constructed as a straight line.

3. A device according to one of Claims 1 or 2, characterised in that the adjustment path (11) intersects the longitudinal axis of the therapy transducer (2).

4. A device according to Claim 1, characterised in that the adjustment path is constructed as a circular ring or circular ring section around the longitudinal axis of the therapy transducer (2).

5. A device according to Claim 1, characterised in that the locating transducer (5) is adjustable by motor along the adjustment path.

6. A device according to Claim 1, characterised in that the locating transducer (5) is rotatable about its longitudinal axis.

7. A device according to Claim 1, characterised in that the locating transducer (5) is axially adjustable.

8. A device according to Claim 1, characterised in that the head of the locating transducer (5) is detachable and interchangeable.

## Revendications

1. Dispositif pour la localisation spatiale et la destruction d'objets à l'intérieur du corps, constitué par un transducteur thérapeutique électro-acoustique (2) à autofocalisation, agencé sous la forme d'une calotte sphérique et servant à produire des ondes de choc devant être émises d'une manière focalisée sur l'objet respectif, et par au moins un scanner B relié au transducteur thérapeutique et agencé sous la forme d'un transducteur de localisation (5) pour la localisation de l'objet, et dans lequel le transducteur de localisation (5) est déplaçable par rapport au transducteur thérapeutique (2) sur une trajectoire prédéterminée et à une distance constante (d'une manière confocale) par rapport au foyer (3) des ondes de choc du transducteur thérapeutique, de manière à pouvoir produire et représenter, en fonction de la position du transducteur de localisation, des images B dans différents plans de coupe et suivant des angles d'observation différents, caractérisé en ce que la trajectoire prédéterminée de déplacement du transducteur de localisation (5) est formée par une ouverture mécanique en forme de fente (11), prévue dans la calotte sphérique du transducteur thérapeutique (2), pour le transducteur de localisation (5) entouré par le champ d'ondes de choc.

2. Dispositif selon la revendication 1, caractérisé en ce que la trajectoire de déplacement pour le transducteur de localisation - vue dans la direction axiale du transducteur thérapeutique dirigé vers la surface d'irradiation - est réalisée sous la forme d'une droite.

3. Dispositif selon l'une des revendications 1 ou 2, caractérisé en ce que la trajectoire de déplacement (11) coupe l'axe longitudinal du transducteur thérapeutique (2).

4. Dispositif selon la revendication 1, caractérisé en ce que la trajectoire de déplacement est réalisée sous la forme d'un anneau circulaire ou d'une section d'anneau circulaire autour de l'axe longitudinal du transducteur thérapeutique (2).

5. Dispositif selon la revendication 1, caractérisé en ce que le transducteur de localisation (5) est déplaçable au moyen d'un moteur le long de la trajectoire de déplacement.

6. Dispositif selon la revendication 1, caractérisé en ce que le transducteur de localisation (5) peut tourner autour de son axe longitudinal.

7. Dispositif selon la revendication 1, caractérisé en ce que le transducteur de localisation (5) est déplaçable axialement.

8. Dispositif selon la revendication 1, caractérisé en ce que la tête du transducteur de localisation (5) est amovible et interchangeable.
